Europäisches Patentamt

(19) European Patent Office

Office européen des brevets

(11) Publication number: **0 174 805**
**B1**

(12) **EUROPEAN PATENT SPECIFICATION**

(45) Date of publication of patent specification: **07.12.88**

(21) Application number: **85306301.4**

(22) Date of filing: **05.09.85**

(51) Int. Cl.⁴: **G 01 N 33/68,** G 01 N 33/53, C 12 P 21/00

(54) **Immunodiagnostic methods.**

(30) Priority: **06.09.84 GB 8422513**

(43) Date of publication of application:
**19.03.86 Bulletin 86/12**

(45) Publication of the grant of the patent:
**07.12.88 Bulletin 88/49**

(84) Designated Contracting States:
**CH DE FR GB IT LI**

(56) References cited:
**DE-A-3 117 725**

**THE JOURNAL OF BIOLOGICAL CHEMISTRY,
vol. 250, March 1975, U.S.A., S. KUWABARA
"Purification and properties of tuberculinactive
protein from mycobacterium tuberculosis", pp.
2556-2562**

**AMERICAN REVIEW OF RESPIRATORY
DISEASE, vol. 126, July 1982, USA, R.G.
BENJAMIN et al. "Serodiagnosis of
Tuberculosis using immunoabsorbent assay
(ELISA) of antibody to mycobacterium
tuberculosis antigen 5", pp. 1013-1016**

(73) Proprietor: **THE WELLCOME FOUNDATION
LIMITED**
**183-193 Euston Road**
**London NW1 2BP (GB)**

(72) Inventor: **Krambovitis, Elias**
**Langley Court**
**Beckenham Kent, BR3 3BS (GB)**

(74) Representative: **Rollins, Anthony John et al**
**Group Patents & Agreements The Wellcome
Foundation Ltd Langley Court
Beckenham Kent BR3 3BS (GB)**

(56) References cited:

**AMERICAN REVIEW OF RESPIRATORY
DISEASE, vol. 123, May 1981, USA, T.M.
DANIEL et al. "Initial clinical trial of
mycobacterium tuberculosis antigen 5 in
tubercullin-positive human subjects", pp. 517-
520**

Courier Press, Leamington Spa, England.

## Description

The present invention relates to immunoassay techniques for use in the diagnosis of mycobacterial infections such as those caused by *Mycobacterium tuberculosis*.

In particular, the present invention relates to a two-part immunoassay system comprising an antigen detection immunoassay and an antibody detection immunoassay, to antigen detection and antibody detection immunoassays employing a novel proteinaceous substance, preferably isolable from *M. tuberculosis*, antibodies to that substance, a novel proteinaceous substance *per se*, a skin test and agglutination tests utilising the substance and/or its antibodies, and diagnostic kits based upon the abovementioned techniques.

Current methods of diagnosis of *M. tuberculosis* infections commonly used include the analysis of clinical symptoms, chest X-rays, staining and microscopic examinations, animal inoculation, culturing and the tuberculin skin test. These methods all suffer from the disadvantages of being either unreliable or time consuming or expensive.

Several workers have developed immunoassay techniques for the diagnosis of tuberculosis; immunoassays can in principle be both inexpensive and rapid. Thus Nassau *et al* (*Tubercle*, 1976, *57*, 67—70) used an enzyme-linked immunosorbent assay (ELISA) for detection of antibodies to mycobacterial culture filtrate in patients with tuberculosis. However, there was considerable overlap in patient and control values. Grange *et al* (*Tubercle*, 1980, *61*, 145—152) reported high ELISA IgG titres in tuberculous patients using crude sonicates of *M. bovis* BCG strain, *M. kansasii* and *M. smegmatis* as antigen but there was inadequate discrimination between infected and control samples, as 20 per cent of tuberculosis patients did not have significantly elevated antibody levels. Tandon *et al* (*Tubercle*, 1980, *61*, 8789), using a partially purified extract of *M. tuberculosis*, Purified Protein Derivative (PPD) as antigen reported that 66 per cent of cases with no evidence of disease as well as 80 per cent of tuberculous patients had detectable antibodies to the antigen. A similar technique but employing radioimmunoassay as well as ELISA has been reported by Zeiss *et al* (*J. Clin. Microbial*, 1982, *15*, 93—96).

PPD, although a partially purified preparation, nevertheless contains a considerable number of proteinaceous and glycolipidic substances. Recently, the isolation, purification and identification of individual proteins and glycolipids from crude mycobacterial extracts has been carried out by a number of workers, see for example Kiyotani *et al* (*Microbiol. Immunol.* 1983, *27*, 695) and Kuwabara (*J. Biol. Chem.*, 1975, *250*, 2556). A few investigators have employed purified mycobacterial proteins and glycolipids in immunoassay techniques, see for example UK Patent Application 2076406. Other examples include that of Reggiardo et al (*J. Immunol. Meth*, 1980, *34*, 55—60) who used three highly glycolipids extracted from *M. bovis* strain BCG in an ELISA assay. Daniel *et al* (*Am. Rev. resp. Dis.*, 1981, *123*, 517—520) used purified mycobacterial proteins and polysaccharides in an ELISA study but they did not include negative controls. Stroebel *et al* (*J. Inf. Dis.*, 1982, *146*, 280—283) using antigen 6 in ELISA found significantly higher antibody titres in patients with active bone tuberculosis than in patients with inactive bone disease or other forms of chronic osteomyelitis. Benjamin and Daniel (*Am. Rev, resp. Dis.*, 1982, *126*, 1013—1016), using antigen 5, reported 95.8 and 79.9% specificity in patients from low and high prevalence areas respectively.

Despite the abovementioned reports, the only commercially available test kit of which the applicants are aware is a latex agglutination kit "agglutinotest®", manufactured by Immunitalia s.r.l. of Pomezia (Rome). This test is designed to detect antibodies to *M. tuberculosis*.

It has been discovered that the combination of an assay for the detection of antibodies to a mycobacterial antigen with an assay for the detection of the mycobacterial antigen provides a clinically useful, rapid effective means of detecting Mycobacterial infections, the sensitivity of the combined assay being significantly greater than that of each of the constituent assays when used singly. Moreover, there have also been discovered a novel antiganic proteinaceous susbtance, the said substance preferably being preparable from *M. tuberculosis* extracts, and antibodies to the antigenic substance, the proteinaceous substance and antibodies being particularly useful in the abovementioned immunoassays and related serodiagnostic tests.

According to one aspect of the present invention therefore, there is provided a method for the detection of mycobacterial infections in a patient, which method comprises the use in combination of an assay for the detection of a mycobacterial antigen with an assay for the detection of an antibody to the mycobacterial antigen.

Assay techniques suitable for use in combination according to the present invention are those known in the art as being useable singly int he detection of mycobacterial antigens or antibodies and include, for example, enzyme immunoassays, radioimmunoassays and agglutination assays.

It has been found most convenient to employ enzyme immunoassays for the purposes of the present invention; in particular an Antigen Capture Immunoassay (ACIA) for the detection of mycobacterial antigen and an Enzyme Linked Immunosorbent Assay (ELISA) for the detection of antibodies to mycobacterial antigens.

Thus in a preferred aspect there is provided a method for the detection of mycobacterial infections in a patient which method comprises the use in combination of an Antigen Capture Immunoassay (ACIA) for the detection of a mycobacterial antigen, and an Enzyme Linked Immunosorbent Assay (ELISA) for the detection of antibodies to the mycobacterial antigen, said ACIA comprising:

bringing a biological fluid sample from a patient into contact with a solid phase to which are bound antibodies to a mycobacterial antigen, adding a conjugate comprising an enzyme bound to an antibody to the mycobacterial antigen, separating the solid phase, adding substrate for the enzyme, determining any enzyme activity on the solid phase and relating this to the presence of mycobacterial antigen in the fluid sample; and said ELISA comprising:

bringing a biological fluid sample from a patient into contact with a solid phase to which is bound an antigenic substance isolable from the mycobacterial species, adding a conjugate comprising an enzyme linked to an antibody to patient immunoglobulin, separating the solid phase, adding substrate for the enzyme, determining any enzyme activity on the solid phase and relating this to the presence in the fluid sample of antibodies to the mycobacterial antigen.

The term "patient" used in the context of the present invention refers to any animal, human or otherwise, which it is desired to test for the presence of a mycobacterial infection. Such mycobacterial infections include those due to *M. tuberculosis, M. Bovis,* and *M. avium.* Preferably the patient is a human.

According to a further aspect of the invention, there is provided proteinaceous substance recognisable by human serum antibodies to *M. tuberculosis* and consisting substantially of proteins of molecular weight in the 5—18 K Daltons, which proteins are immunochemically stabloe at 80°C and within the pH range 5—8, are free of, or substantially free of, lipid or carbohydrate residues and contain less than 0.1% diaminopimelic acid. The proteinaceous substance will be referred to hereinafter as SDS—M.

Preferably, but not exclusively, the proteinaceous substance SDS—M is obtainable from *Mycobacterium tuberculosis* by extraction with sodium dodecyl sulphate. The proteinaceous substance SDS—M may be further purified by gel filtration chromatography followed by ion-exchange chromatography on DEAE cellulose. The further purified substance will be referred to hereinafter as DEAE—II. If so desired, the further purified DEAE—II may be employed as the reagent antigenic substance in any of the diagnostic methods of the present invention as defined above or hereinafter which require the use of such a substance.

The present invention also provides an immunoassay method for the detection of mycobacteria, mycobacterial antigen and antibodies to mycobacterial antigen, the said immunoassay comprising the use, as an immunoreactive component, of the proteinaceous substance as hereinbefore defined, or an antibody thereto. The term immunoassay method includes any method known in the art in which an immunoreactive component e.g. an antibody or an antigen is employed.

In a particular aspect of the present invention, there is provided a method for the detection of antibodies to *M. tuberculosis* in a test sample, which method comprises the steps of a) bringing the test sample containing said antibodies into contact with a solid phase to which is bound the abovementioned proteinaceous substance, b) adding a conjugate consisting of an enzyme linked to a substance selectively bindable to the said antibodies but not to the said proteinaceous substance, c) adding substrate to the enzyme and determining the activity of any enzyme bound to the solid phase.

Preferably, the selectively bindable substance is an antibody to the said antibodies, for example antibody to human immunoglobulin.

In a still further aspect there is provided a method for the detection of antigens of *M. tuberculosis* in a test sample, which method comprises the steps of a) bringing the test sample into contact with a solid phase to which is bound antibody to the abovementioned proteinaceous substance, b) adding a conjugate consisting of an enzyme bound to an antibody to said proteinaceous substance and c) adding substrate for the enzyme and determining the activity of any enzyme bound to the solid phase.

Preferably the antibody forming part of the antibody-enzyme conjugate is a purified antibody.

In yet another aspect of the present invention there is provided a direct agglutination test for the detection of *M. tuberculosis* infections which test comprises bringing a sample of biological fluid from a patient into contact with a suspension of particles coated with SDS—M proteins or antibodies to SDS—M proteins, and determining the presence of *M. tuberculosis* infection by establishing whether or not agglutination of the particles has taken place.

Also within the scope of the present invention are indirect agglutination tests utilising the antigen SDS—M and antibodies to this antigen. Thus there is provided an indirect agglutination test for the detection of *M. tuberculosis* infections, which test comprises the mixing of a biological fluid sample from a patient with an antibody to SDS—M, further mixing with a suspension of particles to which are linked the antigen SDS—M and determining whether agglutination has taken place. There is also provided an indirect agglutination test utilising the same principles as the test described hereinabove except that the biological fluid sample is first mixed with the antigen SDS—M, is then further mixed with a suspension of particles to which are bound antibodies to SDS—M and then it is determined whether agglutination has taken place.

In each of the abovementioned immunoassay methods forming part of the present invention, the solid phase may be formed from any material known in the art to be suitable for such a purpose. Conveniently the solid phase for use in the ACIA and ELISA tests is formed from polystyrene.

The solid phase particles referred to in the description of the agglutination tests conveniently are non-viable bacterial cells, erythrocytes, latex polymer beads or any other materials known to the skilled man, see for example UK Patent Application 2076406.

Solid phases to which the abovementioned proteinaceous substance or an antibody thereto, have been bound, are within the scope of the invention.

3

In the Antigen Capture Immunoassay, non-specific interactions, which lead to a decrease in sensitivity of the method, may be eliminated or significantly reduced by coating or "blocking" the antibody coated solid phase with bovine serum albumen (BSA) Typically this is achieved by incubating the antibody-coated solid phase with a solution of BSA, for example a 1% solution, for an appropriate time, 2—3 hours usually being sufficient although longer or shorter periods may be employed if desired.

Coating or "sensitising" of the solid phase with antigen or antibody is carried out according to methods well known in the art.

It has been found most convenient to attach the antigen or antibody to the solid phase by incubating the solid phase with a solution of the antigen or antibody for an appropriate time e.g. 0.5—24 hours at an appropriate temperature e.g. 15°—37°C.

The sensitised solid phase may be prepared and used immediately or it may be stored and used at a later date. To preserve the activity of the immunologically active substances during storage, it has been found convenient to overlay the sensitised solid phase with a coating of degraded gelatin.

Test sera will normally be diluted with an appropriately buffered solution before assaying. Typically a sodium phosphate buffer will be employed to maintain a pH of 7—8.0; most conveniently the immunoassay will be carried out at pH 7.4.

It is preferred that the diluent solution contains substances capable of inactivating factors such as complement and Rheumatoid Factor (RF) which interfere with the immunochemical reaction and thereby cause a reduction in sensitivity of the method. EDTA or salts thereof, which inactivate Complement, and dithiothreitol, which deactivates Rheumatoid Factor are examples of such substances.

The enzyme comprising part of the enzyme-antibody conjugate can be any enzyme known in the art as being useful for the purpose in similar enzyme immunoassays. Enzymes commonly used include peroxidase, β-galactosidase and alkaline phosphatase. It has been found most convenient for the purposes of the present invention to use a conjugate containing peroxidase. The enzyme antibody conjugates *per se* form a further aspect of the invention.

The activity of the peroxidase is determined by the addition of the substrate hydrogen peroxide, and a separate chromogen such as 5-aminosalicylic acid, tetramethylbenzidine or o-phenylenediamine which can react with the initial products of the enzyme reaction.

A further application of the antigenic proteinaceous substance of the present invention is its use in a skin test for the diagnosis of tuberculosis or the detection and identification of antibodies induced by mycobacterial infection. A typical skin test (Mantoux test) involves intradermal injection of a suitable volume (e.g. 0.1 ml) of an appropriate composition of the standardised proteinaceous substance into the most superficial layers of the skin (usually the skin of the forearm in humans). The average diameter of induration is measured after a suitable time (e.g. 72 hours in humans) and reactions less than 5 mm in diameter are recorded as doubtful. If desired, a second test may be carried out on negative or doubtful patients using a more concentrated sample of the antigen.

Compositions for intradermal injection, in addition to containing the abovementioned proteinaceous substance and a carrier, usually water, may also contain other accessory ingredients, for example an isotonic agent such as sodium chloride.

In another embodiment of the invention, there is provided a kit for use in an immunoassay method, the kit comprising, as an immunoreactive component, the proteinaceous substance as hereinbefore defined and/or an antibody or antibodies thereto.

In particular there is provided a kit for use in the detection of antibodies to *M. tuberculosis* by the ELISA method, the kit comprising:

1. a solid phase sensitised with SDS—M or DEAE—II
2. a buffered diluent containing ETDA and dithiothreitol
3. an enzyme linked to an antibody to IgG
4. substrate and chromogen system for the enzyme

There is also provided a kit for the detection of *M. tuberculosis* antigens by the ACIA method, the kit comprising:

1. a solid phase sensitised with antibodies to SDS—M or DEAE—II
2. a buffered diluent containing EDTA
3. an enzyme linked to an antibody to SDS—M or DEAE—II
4. substrate and chromogen system for the enzyme

Other components which can usefully be included in the abovementioned kits include concentrated wash fluid (Tween 20 saline solution (TS)).

The substrate/chromogen system typically comprises freeze dried tetramethylbenzidine as the chromogen with a separate diluent containing a buffering agent, such as sodium acetate or citrate, and the substrate hydrogen peroxide. The enzyme antibody conjugate may be provided in the freeze dried state along with a suitable diluent containing for example Tween 20, phosphate buffering agent and bovine serum albumen (BSA).

The abovementioned kits may be provided for separate use or they may be provided for use in combination.

There is further provided a direct agglutination test kit for the detection of *M. tuberculosis* antigens or antibodies comprising a suspension of latex particles coated with the protein fraction of the present invention or antibodies thereto.

In particular, there is provided a direct latex agglutination test kit for the detection of mycobacterial antigens, the said kit comprising a) latex particles coated with antibodies to SDS—M or DEAE—II, a control latex and a positive control antigen.

The control latex reagent contains latex particles sensitised with either the IgG fraction taken from the animal in which the antibodies to SDS—M or DEAE—II are raised prior to inoculation with the antigen, or the IgG fraction taken from another non-inoculated animal of the same species that has been raised in identical conditions, or a monoclonal antibody of the same class as that used on the test latex but having a different specificity.

A negative control "antigen" solution (saline) can also usefully be included in the kit.

The present invention will now be illustrated by means of examples.

Example 1
Isolation and purification of an antigen from M. tuberculosis

Culture and harvest of Mycobacterium tuberculosis

Freeze dried cultures of *Mycobacterium tuberculosis* were reconstituted with 0.2 ml Dubos medium and transferred to Lowenstein-Jensen slopes. They were then incubated at 37°C for 4—5 weeks. Lowenstein-Jensen slopes which were visually free of contamination were harvested by washing off the surface growth with 5 ml Sauton medium which was then used to inoculate 1-litre bottles containing 500 ml Sauton medium. This was incubated *in situ* at 37°C until the growth was visually satisfactory. The organisms were killed by adding 90% (w/v) phenol to a final concentration of 5% and incubating for a further week at 37°C. The viability of the phenolised organisms was checked by transferring two 0.5 ml of aliquots of cell suspension to fresh Lowenstein-Jensen slopes and incubating at 37°C for 2 weeks. All the work was carried out in a safety cabinet (Microflow), loops and metal instruments were sterilised in a Bacticinerator (Sherwood), and pipettes, gloves and all surfaces were sterilised with 2% glutaraldehyde (Sonacide).

*M. tuberculosis* organisms (strain $H_{37}RV$) were also grown in a 15-litre fermentor (1601) (Ultroferm, LKB) containing 10 litres Sauton medium at 37°C with cosntant supply of 5% $CO_2$—95% air at a rate of 0.5 l/min and with continuous agitation at 300 r.p.m. They were harvested by draining the culture directly into a 20-litre bottle containing phenol calculated to give a final concentration of 5%. The fermentor vessel was then re-filled with fresh medium. There were enogh organisms left in the fermentor to continue the growth without further inoculation. As many as six harvests were obtained in this way with no apparent loss of antigenicity by the organisms. For each series, the first growth required 4 weeks incubation before it could be harvested whereas subsequent growth was rapid and the organisms were harvested every 48—72 hours. During the growth period, the pH was maintained metabolically at 7.2 by titration with 50% (w/v) sterile glucose. From each harvest 20—50 ml of packed cells were obtained.

All phenolised cultures were washed three times (total 30 volumes) with 0.5% phenol in isotonic saline and stored as 10% (v/v) suspension at 4°C.

Staining

The cultures were checked for purity by acid-fast staining (Ziehl-Neelsen's stain). Smears were heat-fixed on a microscope slide (pre-cleaned with alcohol). The slide was then flooded with hot carbol fuchsin solution (1% basic fuchsin in 10% aqueous ethanol containing 5% phenol) for 20 min. After a brief wash in running tap water it was decolourised in acid alcohol (3% hydrochloric acid in ethanol) until no more stain leaked out. It was counterstained with 0.5% methylene blue in distilled water for 5 min., washed in running tap water and dried with blotting paper. The stained organisms were examined under oil immersion in a Leitz microscope at ×900 magnification.

Extraction of Antigens

Extraction of whole cells with sodium dodecyl suphate (SDS)

Mycobacteria (10% v/v) were washed three times in 66 mM phosphate buffer pH 7.4 (PB) to remove phenol and were extracted with 1% SDS in PB at 80°C for 1 hour. After centrifugation at 10,000 g for 1 hour the supernatent was filtered with a 0.22 μ-pore size membrane (Millipore) and chromatographed according to Karp and Vinogradov (*Anal. Biochem.*, 1978, *91*, 230—235) to remove excess detergent. For this 0.5 g of ion retardation resin AG IIa8 (Bio-Rad Laboratories) per ml extract was washed with 5 volumes of 1M ammonium chloride and then with 20 volumes distilled water. The resin was packed into a a small chromatographic column (1 × 10 cm) and loaded with the extract. Elution was carried out at a flowrate of 60 ml/hr (2112 Redirac fraction collector, LKB) and the fractions containing protein, as monitored using a flow cell at 280 nm, were pooled and stored at −20°C. This partially purified protein containing fraction was labelled SDS—M.

Separation of antigenic components by gel filtration

Extracts to be chromatographed were concentrated before filtration using a B15 macrosolute concentrator (Amicon). Alternatively the extacts were concentrated by precipiting with ammonium sulphate and then redissolving the precipitate in phosphate buffered saline containing 0.1% (w/v) SDS.

Chromatography was carried out in either 13 × 1000 mm or 17 × 1000 mm glass columns packed with Sephacryl®-S300 (Pharmacia), which had been pre-equilibrated with 66 mM sodium phosphate buffer pH 7.4.

The sample applied was 3—5% (v/v) of the bed volume, which was then fractionated at a flow rate of approximately 2 ml/cm²/hr (See Figure 1). The protein content of fraction was monitored at 280 nm (Uvicord S, LKB,), recorded (2210 1-channel recorder, LKB), and 1 ml or 2 ml fractions were collected (2112 Redirac fraction collector, LKB). The carbohydrate content was monitored by the phenol-sulphuric acid assay method of Dubois *et al., (Anal. Biochem.,* 1956, *28,* 350—356).

FIGURE 1

Profile of SDS—M fractionated by gel filtration on Sephacryl® S—300(_____antigenic

activity as determined by direct ELISA;_____protein;.......carbohydrate).

FIGURE 2

Profiles of SDS—M fractionated by gel filtration on Sephadex® G—75, G—100, G—200

and Sepharose 6B (————protein profile;————— profile of antigenic activity).

Antigenic activity was checked by direct ELISA using a peroxidase-labelled rabbit anti-SDS—M conjugate, and by gel diffusion.

Two main peaks were observed. The first peak was predominantly protein and had the highest antigenic activity, whilst the second peak which appeared to contain more carbohydrate was much less active.

Similar profiles were obtained with Sephadex® G75, G100 and Sepharose 6B (Figure 2).

Further purification of the S—300 main peak by ion-exchange chromatography.

The major peak obtained by gel filtration was purified further by ion exchange chromatography on DEAE cellulose (DE52, Whatman). Equilibration of the exchanger was adapted from the manufacturer's instructions with certain modifications. DEAE cellulose powder was hydrated by suspending in 5 ml/g exchanger of 1M Tris-HCL pH 8.4 buffer to be converted to salt form. After filtration using Whatman No. 54 filter paper, it was resuspended in 100 mM buffer at 3 ml/ml exchanger, stirred gently for 15 min and then filtered. This cycle of stir-filter-resuspend was repeated twice and then three times in 10 mM buffer. Finally the exchanger was resuspended in 10 mM buffer at 1.5 ml/ml echanger and used to pack a short column by running through two bed volumes of buffer. Fractions from the S—300 filtration of SDS—M containing the antigen were pooled and dialysed overnight against the same buffer.

After application of the sample, the column was washed with two bed volumes of buffer. Over 90% of the antigenic activity was retained on the column. The fraction which did not bind (DEAE I) contained mostly carbohydrate with a small amount of protein. The antigenic activity as detected by direct ELISA was low.

Bound antigen (DEAE II) was eluted as a sharp peak at 0.3 M sodium chloride by applying a linear 0—1 molar gradient of sodium chloride in the same buffer. (See Figure 3).

FIGURE 3

Profile of the S—300 main peak purified by ion—exchange chromatography using DEAE—cellulose (_____antigenic activity as determined by direct ELISA; - - - - - protein: - - - - - carbohydrate).

Arrow indicates the beginning of the sodium chloride gradient.

DEAE II was highly antigenic and appeared to be a protein. No. detectable carbohydrate or free fatty acids were found (less than 0.1% (w/w). The specificity of DEAE II antigen was very similar to that of SDS—M extract when compared by TB—ELISA.

8

## Appendix

Media
Lowenstein-Jensen medium — Media Production Unit, Wellcome Research Laboratories
Dubos medium— Media Production Unit, Wellcome Research Laboratories

Sauton synthetic medium:

| | |
|---|---|
| L-Asparagine | 4.0 g |
| Citric Acid | 2.0 g |
| $KH_2PO_4$ | 0.5 g |
| $MgSO_4.7H_2O$ | 0.5 g |
| Ferric ammonium citrate | 0.05 g |
| Glycerol | 40 ml |
| Tween® 80 (Difco)-detoxified* | 0.2 ml |
| Distilled water | 960 ml |

The pH was adjusted to 7.2 with 1M KOH, and the medium was dispensed into bottles and autoclaved at 121°C for 15 min.

*Detoxification of Tween 80
One part of Tween® 80 was mixed with 5 parts of extraction mixture (isopropanol 40 parts: n-heptane 10 parts: 0.5M sulphuric acid 1 part), shaken vigorously for 5 min, and then allowed to stand for 5 min. Two parts of n-heptane and 12 parts water were then added, mixed thoroughly and allowed to stand until phase separation was complete. The upper phase was removed and discarded while the lower phase was transferred to a small beaker and the remaining isopropanol was evaporated by heating at 80°C.

### Example 2

Studies on the physico-chemical characteristics of SDS—M and DEAE II
Determination of the isoelectric point.
The isoelectric points (pl) of SDS—M and DEAE II were determined by isolectric focussing in polyacrylamide gel (PAG plates pH 3.5—9.5, LKB). Samples and markers were focussed with constant power of 30 Watts for 1.5 hours.
Both SDS—M and DEAE II stained weakly with Coomassie blue, were P.A.S. (H. Glossman and D. M. Neville, *J. Biol. Chem.*, 1971, *246*, 6339—6346) negative, but counterstained with ANS (H. Glossman and D. M. Neville, *J. Biol. Chem.*, 1971, *246*, 6339—6346) strongly. The pl of SDS—M was 3.5 and of DEAE II 3.4, both of which were very close to the limit of the pH range of the ampholytes.

Polyacrylamide gel electrophoresis studies in the presence of SDS (SDS-Page)
Experiments with SDS—M conducted in gel rods using the SDS-phosphate continuous buffer system (Weber and Osborn, *J. Biol. Chem.*, 1969, *244*, 4406—4412) showed that the antigenic activity as determined by ELISA from gel section extracts was confined to two bands. The more specific band, which stained with Coomassis blue and was P.A.S. positive, had an Rf value of 0.95 (±0.3) and an estimated molecular weight of 12.,4 (±0.52) K daltons. In a two-dimensional polyacrylamide to agarose immunoelectrophoresis experiment two immunoprecipitin lines were obtained. These lines corresponded to the active bands.
Using a 4 to 30% polyacrylamide gradient gel (Pharmacia) in 40 mM Trisacetate-ADS buffer pH 7.4 the smaller component could be separated to two further components. The slower of these was P.A.S. positive only and corresponded to DEAE I, whereas the faster component was a P.A.S. negative polypeptide which corresponded to DEAE—II.

Determination of molecular weights.
Molecular weights were determined by subjecting SDS—M and DEAE II to electrophoresis in parallel with standard proteins on 10% gels in tris-glycine discontinuous buffer system (Laemmli, *Nature,* 1970, *227,* 680—685). Over the range 30—60 K daltons four weak proteins were identified in SDS—M which were absent in DEAE II. The resolution of proteins and peptides of molecular mass less than 20 K daltons was, however, poor because they remained stacked at the moving boundary.
The molecular weights of the peptides of molecular weight less than 20K Daltons were resolved using 12.5% gels in a urea-tris-phosphate buffer system (Swank and Munkres, *Analyt. Biochem.,* 1971, *39,*

462—477). Fraction DEAE—II consisted of a family of five peptides ranging between 5.5 and 17 K daltons. Of these the main one had a molecular weight of 12 K daltons whereas the other four were only weakly stained with Coomassie blue. The estimated molecular weights of the protein and peptide components of SDS—M and DEAE II as determined by the two methods are summarised in table 1.

TABLE 1

| Buffer system | Acrylamide Molecular weight (K daltons) | Antigen | |
|---|---|---|---|
| | | SDS—M | DEAE II |
| Laemmli (1970) | 10% (0.6% C) | 66 | — |
| " | " | 58 | — |
| " | " | 46 | — |
| " | " | 34 | — |
| Swank & Munkres | 12.5% (6.8% C) | 15 | 17 |
| " | " | 12 | 12 |
| " | " | 9 | 8.4 |
| " | " | 6.7 | 6.4 |
| " | " | 5.8 | 5.8 |

Determination of the amino acid composition of DEAE II

The amino acid composition of DEAE II was determined according to standard methods. The results are shown in table 2.

TABLE 2

Amino acid composition of purified DEAE II from *Mycobacterium tuberculosis* $H_{37}RV$.

| Amino acid | Concentration nMol/ml | No. of residues Per molecule** |
|---|---|---|
| Lysine | 3.16 | 5 |
| Histidine | 2.65 | 4 |
| Arginine | 4.79 | 7 |
| Aspartic acid | 7.67 | 11 |
| Threonine | 5.39* | 8 |
| Serine | 4.68* | 7 |
| Glutamic acid | 8.80 | 16 |
| Proline | 5.92 | 8 |
| Glycine | 8.98 | 13 |
| Alanine | 11.41 | 16 |
| Valine | 7.77 | 11 |

TABLE 2 (continued)

| Amino acid | Concentration nMol/ml | No. of residues Per molecule** |
|---|---|---|
| Methionine | 2.87 | 4 |
| Isoleucine | 3.43 | 5 |
| Leucine | 7.06 | 10 |
| Tyrosine | 1.39* | 2 |
| Phenylalanine | 2.13 | 3 |
| Tryptophan | N.D. | — |
| Diaminopimelic acid | 0 | 0 |

** the values were calculated and adjusted to the best whole number by assuming that the molecular weight of DEAE II was 12, K dalton. Except for threonine, serine and tyrosine, each value was the average obtained by hydrolysis for 24, 40 and 72 hours.
* values extrapolated to zero time of hydrolysis
N.D. not determined

Analysis of the Physical Properties of DEAE II

The antigen DEAE II was treated under a variety of conditions with a variety of reagents and the level of antigenic activity remaining after treatment was determined by direct ELISA. The results are listed in Table 3.

TABLE 3
Physical properties of DEAE II

| Treatment* | % Residual activity |
|---|---|
| Trypsin | 0 |
| Pronase | 0 |
| DNAase | 100 |
| RNAase | 100 |
| Phosphorylase A | 100 |
| Lipase | 100 |
| Dextranase | 100 |
| Neuraminidase | 100 |
| 0°C | 100 |
| 25°C | 100 |
| 37°C | 100 |
| 80°C | 100 |
| 100°C | 60 |
| pH 5—8 | 100 |
| pH <3 | 0 |
| pH >10 | 0 |

* DEAE II was incubated for 1 hour with each treatment.

11

## Example 3

Production of antisera

(i) Antibodies to SDS—M or DEAE—II for use in the ACIA and direct ELISA assys were prepared as follows:

New Zealand white rabbits weighing approximately 2.5 kg were immunised with 1—5 mg/ml antigen emulsified with an equal volume of Freunds incomplete adjuvant 0.1 ml was injected into the hind limb muscles at several sites. The rabbits were challenged 7 and 14 days later with a similar dose. 40 ml blood samples were taken 4, 6, 8 weeks after the final challenge and the resultant serum was stored at −20°C.

(ii) Antihuman immunoglobulins for use in the antibody detection indirect ELISA were raised in sheep according to standard procedures.

Preparation of conjugate

Immunoglobulins required for enzyme labelling and for certain immunoprecipitation studies were purified from whole serum substantially according to the method of Axelsen, (*Infect. Immun.*, 1971, *4*, 525—527). Conjugation of horseradish peroxidase to the immunoglobulins was performed using dimaleimides as described by Duncan, *et al.*, (*Analyt. Biochem.*, 1983, *132*, 68—73).

For long-term storage the conguates were lyophilised in 10 mg/ml mannitol and 10 mg/ml bovine serum albumin. For short-term storage, they were kept in 50% glycerol at 4°C.

## Example 4
### Enzyme Immunoassays

Indirect enzyme immunoassay (TB—ELISA)

Volumes of 200 µl per well were used throughout the assay. Microtitre plates (Titertek Flow Laboratories) were sensitised with 30—50 µg/ml antigen (SDS—M or DEAE II) in 0.06M phosphate buffer pH 7.4 (PB) at 37°C for 30 min. Excess antigen was washed off with 0.05% Tween® 20 (Koch-Light Laboratories) in saline (TS) for 10 minutes. Test sera were diluted in PB containing 1 mM dithiothreitol, 11 mM EDTA, and 0.05% Tween 20 and added to the sensitised wells. They were incubated at room temperature for 1 hour and the wells were washed for 10 min with TS. The appropriate conjugate was diluted to its optimum working strength with PB containing 0.5% bovine serum albumin and 0.05% Tween® 20 (conjugate diluent) and added to the wells. This was incubated at room temperature for 60 min and washed with 8 changes of TS over 15 min. The substrate-chromogen solution was prepared just before use by dissolving 0.8 mg/ml 5′-aminosalicylic acid (Cambrian Chemicals) purified and recrystallised according to the method of Ellens and Gielkens (*J. Immunol. Meth.*, 1980, *37*, 325—332) in 0.05 M citrate buffer pH 6.5 containing 0.2 µl 8.8 M hydrogen peroxide per ml. The substrate-chromogen was added to the wells and incubated or 30 min at room temperature. The reaction was stopped by adding 25 µl 1 M NaOH per well and the absorbance was measured at 450 nm (Titertek multiscan, Flow Laboratories). A diagrammatic representation of the assay is shown in Fig. 4.

Direct ELISA

Microtitre plates were sensitised with 200 µl of antigen as above and washed with TS for 10 minutes. The appropriate conjugate diluted to its working strength with conjugate diluent was added in 200 µl aliquots per well. It was incubated at room temperature for 60 min and washed with TS for 15 min. Substrate-chromogen was added to the wells and incubated for 30 mins at room temperature. The reaction was stopped with sodium hydroxide and the absorbance of each well was measured at 450 nm. A diagrammatic representation of the assay is shown in Fig. 5.

Antigen Capture Immunoassay (ACIA)

Volumes of 200 µl per well were used throughout the assay unless stated otherwise. Purified anti-mycobacterial immunoglobulins were diluted with 50 mM carbonate buffer pH 9.5 to 150 µg/ml and added to the wells of a microtitre plate. The plates were sensitised at 37°C for 3 hr and then stored at 4°C until used.

Test samples were diluted 1:5 with 100 mM EDTA (pH 7.4) in PB and heated at 80° for 10 min. The denatured material was centrifuged at 3,000 g for 15 min and the resultant supernatant was dialysed for 2 hr in a large volume of PB.

A sensitised plate was washed with TS for 10 min to remove unbound antibody. Sample supernatants and dilutions of a standard antigen solution were placed into appropriate wells. The plate was incubated at 37°C for 3 hour and washed with TS for 10 min. Peroxidase-labelled rabbit anti-mycobacterial IgG was diluted to working strength (approximately 20 µg/ml) with conjugate diluent and added to each well. The plate was incubated at room temperature for 1 hr and washed with 8 changes of TS over 15 min.

The substrate-chromogen solution (as above) was added to the wells and incubated for 30 min at room temperature. The reaction was stopped with 25 µl sodium hydroxide and the absorbance of reference antigen dilutions, and the antigen content of positive samples was estimated. A diagrammatic representation of the assay is shown in Fig. 6.

Fig. 4. Diagrammatic representation of the indirect enzyme immunoassay (TB—ELISA) for measuring antibody activity in human sera.

TB antigen

Peroxidase labelled
rabbit anti—TB

Substrate + Chromogen

Enzyme reaction product (Δ)

Fig. 5. Diagrammatic representation of the direct enzyme immunoassay (direct ELISA)
for measuring antigen activity.

Fig. 6. Diagrammatic representation of the antigen capture immunoassay (ACIA) for detecting antigen in human sera.

Example 5
Evaluation of the TB-ELISA using sera from London Hospital.

Serum samples were collected from patients who were either hospitalised or attending the outpatients clinic of the Chest Department at the London Hospital. Diagnosis was made by expert hospital clinicians according to standard criteria before a sample was taken. The sera were tested for hepatitis B virus using "Hepatube" (Wellcome Diagnostics), randomised, coded and stored at −20°C until use. They were tested blind.

The patients studied were classified into six tuberculosis groups and six control groups, four of which being associated with other lung diseases would make it desirable for the assay to discriminate them from pulmonary tuberculosis. The results obtained from the tuberculosis patients are summarised in figure 7, from which it can be seen that if activity of 50 was taken as the cut-off point, then the assay was 76.5 per cent sensitive with new pulmonary tuberculosis cases and 84.5 per cent with extra-pulmonary cases. All relapsed patients and also the three cases with non-tuberculosis mycobacterial infections were also positive. Half of the cases with quiescent tuberculosis were negative whereas the others were marginally positive.

The controls were all sera from patients with other diseases. These were divided into three main groups. The first group comprised patients suspected of pulmonary tuberculosis because of abnormalities in their chest X-ray but which were proven to be otherwise. All samples gave reactions less than 50 (Fig. 8). The second group contained patients with other lung associated diseases. Reactions from the sera of patients with lung carcinoma, sarcoidosis and chronic bronchitis were below 50, and only one case in the pneumonia group gave a reaction of more than 50.

The efficiency of the TB-ELISA relative to other techniques used in clinical laboratories to diagnose TB is shown in table 4.

FIGURE 7

ELISA Determination of Antibody Activities of

Patients with Mycobacterial Infections

16

EP 0 174 805 B1

Fig. 8

ELISA Determination of Antibody Activities in Control Patients

Exhibiting Abnormal Chest X—rays or having Lung Associated Diseases.

TABLE 4

Performance characteristics of the criteria used to diagnose tuberculosis infections at the London Hospital as compared with the clinical ability of the TB-ELISA test.

|  | Predictive Value | | |
|---|---|---|---|
|  | Positive Value | Negative Value | Efficiency |
| Chest X-ray | 0.44 | 0.85 | 0.60 |
| Chest X-ray and clinical status | 0.67 | 0.79 | 0.71 |
| Microscopical examination of sample | 0.88* | 0.60 | 0.725 |
| Enzyme immunoassay (TB-ELISA) | 0.85 | 0.85 | 0.86 |
| Culturing of sample | 1.00 | 0.85 | 0.89 |

*Including the non-tuberculosis mycobacteria

Example 6

The detection of TB antigen in sera from patients with natural infection using the ACIA Method.

Serum samples were obtained from 70 London Hospital patients, 30 of whom were pre-treated TB cases and the other 40 of whom were non-TB control patients. The sera from each of these patients were treated according to the method of Smith et al (Abstracts of the annual meeting of Am. Soc. Microbiol., 1981, c140) in order to dissociate the antigen from its immune complex. Thus test serum was diluted 1:4 with sodium EDTA (0.1M) at pH 7.4, heated at 80°C for 10 minutes, centrifuged to sediment denatured material and the resulting supernatant was then dialysed for 1—2 hours against sodium phosphate buffer (pH 7.4). The dialysate was used immediately in the ACIA test. ACIA tests were carried out blind according to the method described in Example 4 using anti-SDS-M as coating antibody and conjugate. Serial concentrations of antigen SDS-M were added to a negative serum and were extracted and assayed in order to determine a standard curve.

The frequency distribution of ACIA values for the control group (Fig. 9) had a sample median of 0.14 and a range of 0.08 to 0.20. It was assumed that this group consisted of individuals totally lacking in tuberculosis antigen. Optical density of 0.22 was chosen as the baseline for positivity. This value was the mean (0.147) plus three standard deviations (D.0.027) of the ACIA values of the control group.

Thirteen (43%) of the tuberculosis samples were ACIA positive (Fig. 10), four of which contained antigen concentrations of 10 μg/ml or higher. When the results of ACIA were combined with those of the TB-ELISA (Fig. 11) 28 samples (93%) were positive in either or both tests. Only two sera, one from a pulmonary and one from a genitourinary case were negative in both assays.

The combined results of TB-ELISA and ACIA assays gave an improved predictive value (Table 5) and consequently improved efficiency over the single assay for antibody detection.

Fig. 9. Frequency distribution of ACIA readings for 40 control patient sera.

Fig. 10. Distribution and concentration of detected antigen activity by ACIA in thirty tuberculosis and forty control patients sera after heat treatment.

Fig. 11. Comparison of ACIA positive and negative sera in relation to their respective antibody activity as detected by TB—ELISA.

TABLE 5

Performance characteristics of the antigen detection (ACIA) and antibody detection (TB-ELISA) tests.

| Assay | Sensitivity | Specificity | Predictive value | | Efficiency |
|---|---|---|---|---|---|
| | | | Positive | Negative | |
| ACIA | 0.43 | 1.00 | 1.00 | 0.30 | 0.76 |
| TB ELISA | 0.73 | 0.87 | 0.81 | 0.81 | 0.81 |
| ACIA+TB ELISA | 0.93 | 0.87 | 0.85 | 0.94 | 0.90 |

EP 0 174 805 B1

<div align="center">

Example 8

Rapid Detection of TB antigen by Latex Agglutination

</div>

Sensitisation of latex and test procedure

Polystyrene latex particles were non-covalently coupled to anti-SDS-M purified immunoglobulins for use in the detection of TB antigen in body fluids. Latex suspension with polystyrene particle size of 0.173 μ in diameter (Dow Laboratories) was diluted to 1% (w/v) with 0.1M glycine buffered saline pH 8.2. This was passively sensitised by adding an equal volume of antibody solution and incubating the latex-antibody mixture at 56°C for 25 min. Optimum sensitivity was obtained when 10 mg of immunoglobulin were offered per gram latex (dry weight). A control latex suspension was prepared by the same method using immunoglobulins from rabbits not exposed to TB antigens.

The test procedure comprised mixing 50 μl of body fluid sample (previously heated to 56°C for one hour to remove non-specific interference) with 25 μl of test latex or control latex on a black plate followed by rocking the plate for 2 minutes.

A positive reaction was indicated by the development of an agglutination pattern. The speed of the appearance and quality of latex agglutination seemed to depend on the strength of the antigen present in the sample and varied from large clumps which appeared within a few seconds of mixing, to smaller clumps which developed rather slowly. Clear agglutination of the test latex accompanied by a lack of agglutination of the control latex was interpreted as an indication of the presence of TB antigens in the body fluid. Visible agglutination of the control latex indicated that the reaction was non-specific.

Latex agglutination studies with cerebrospinal fluids (CSF)

Detection of mycobacterial antigens by the latex reagent was evaluated in two studies, (i) testing CSF samples from children and (ii) testing CSF samples from adults.

(i) Tuberculous meningitis (TBM) in children.

A collaborative study was undertaken with Great Ormond Street Hospital for Sick Children. Investigations were carried out on samples of CSF obtained from eighteen children with tuberculous meningitis (TBM). Seven of these children were recent patients from whom fresh samples were available. The remaining eleven patients were those identified as cases of TBM by a search of medical records from a ten year period and in whom stored cerebrospinal (CSF) was available. TBM was diagnosed on the basis of findings in the CSF. In fifteen cases this was by the demonstration of acid-alcohol fast bacilli (AAFB) in films made from centrifuged CSF deposit, and where these were not observed, by cytological and biochemical findings compatible with the diagnosis of TBM, supported by clinical and epidemiological evidence.

Control data were obtained by the examination of 134 fresh CSF samples sent to the Department of Microbiology with diagnoses other than TBM (table 6). Thirty eight samples were from children with acute pyogenic meningitis; the remaining ninety six were from patients with a variety of infective and non-infective conditions in whom the diagnosis of TBM was not considered to be appropriate and subsequent clinical and laboratory findings supported this differentiation. TB antigen was detected in 17 of 18 cases on initial testing. In the remaining case the initial sample was negative but subsequent samples, including one taken at post mortem, gave positive results.

Tests carried out on samples from the control group gave negative results with 133 of 134 investigated (table 6). A positive result was obtained with a sample from a child with *H.influenzae* type b meningitis and subdural abscess formation. No pathogens were isolated from this CSF which gave a strong reaction with *H.influenzae* type b latex (Wellcogen *H.influenzae b*, Wellcome Diagnostics) as well as the TB latex reagent. Both of these reactions were inhibited specifically by addition of homologous antiserum to the CSF prior to test (table 7).

All of the CSF samples were retested in a blind trial by another operator and in all cases, test as well as control, the same results were obtained.

The findings of this study illustrate the usefulness of the latex particle agglutination assay of the present invention in the diagnosis of tuberculosis meningitis.

<div align="center">

TABLE 6

Control Patients tested for TB antigen in CSF

</div>

| Control Group | No. Tested | No. Positive |
|---|---|---|
| Bacterial meningitis | 38 | 1 |
| (*Haemophilus influenzae b*) | (18) | (1) |
| (*Streptococcus pneumoniae*) | (12) | (0) |
| (*Neisseria meningitidis*) | (6) | (0) |
| (*Streptococcus Group B*) | (2) | (0) |

<div align="center">

21

</div>

TABLE 6 (continued)
Control Patients tested for TB antigen in CSF

| Control Group | No. Tested | No. Positive |
|---|---|---|
| Acute leukaemia | 28 | 0 |
| Febrile illness | 19 | 0 |
| Neurodegenerative disease | 14 | 0 |
| Hydrocephalus | 11 | 0 |
| Encephalitis | 8 | 0 |
| Myelogram | 5 | 0 |
| Febrile convulsion | 5 | 0 |
| Glioma | 3 | 0 |
| Head injury | 2 | 0 |
| Near miss cot death | 1 | 0 |
| TOTALS | 134 | 1 |

TABLE 7
Cross-inhibition studies on a false-positive control CSF sample

| Blocking Antibodies | Result with *H.influenzae b* reagent | Result with TB reagent |
|---|---|---|
| Anti-*M.tuberculosis* plasma membrane | + | − |
| Anti-*H.influenzae* type b capsular antigen | − | + |
| *No Antibodies added* | + | + |
| Anti-*H.influenzae* type b capsular antigen and anti-*M.tuberculosis* plasma membrane | − | − |

+ = Agglutination obtained
− = No agglutination.

(ii) Latex tests with adult CSF's

Twenty fresh CSF's from meningitis patients were obtained from Lewisham Hospital, and tested blind. Nineteen of these were negative with the latex test. One sample was positive. This sample originated from a TBM suspected patient, and a second sample a week later also confirmed the earlier positive result. Presence of acid fast bacilli was reported in the second CSF but not the first. The case was confirmed as TB two weeks later, by positive culture.

Example 9

8.4 Guinea pig skin test reactivity of DEAE II

The skin test reactivity of DEAE II was studied and compared with that of purified protein derivative (PPD) in guinea pigs.

Groups of two guinea pigs were sensitised by a single intramuscular injection of 0.05 mg phenol-killed mycobacteria in Freund's incomplete adjuvant (Wellcome Research Laboratories). Each group of animals was injected with (1) *M.tuberculosis* strain $H_{37}RV$, (2) *M.tuberculosis* strain $H_{37}RA$, and (3) *M.xenopi.*

The guinea pigs were skin tested four weeks after sensitisation. DEAE II and PPD were dissolved and diluted in physiological saline without addition of Tween 80. Graded doses of antigen (0.1 ml) were injected intradermally on a shaved flanks. The mean diameter of induration was measured 24 hr after injection. PPD used in the experiments was prepared from *M.tuberculosis* strain $H_{37}RV$. It was obtained from the National Institute of Allergy and Infectious Diseases (Bethesda, USA), and the stated potency was 30,000 international Units per mg.

The results, which are summarised in Table 8, show that DEAE II reacted only in guinea pigs sensitised with *M.tuberculosis*. There were no skin reactions with *M.xenopi* except at the highest dose which occurred also with PPD. The potency of DEAE II in the skin reaction was about 10 times stronger than that of PPD. The time course of the reaction appeared within 3 hours after injection.

TABLE 8

Skin test reactivity of DEAE II and PPD in guinea pigs with two species of mycobacteria.

Diameter of induction (mm) 24 hr after injection
in guinea pigs sensitised with phenol killed cells of

| Antigen | Protein concentration | *M.tuberculosis* $H_{37}RV$ | *M.tuberculosis* $H_{37}RA$ | *M.xenopi* | Control |
|---|---|---|---|---|---|
| DEAE II | 100 | 18.3 ± 1.9 | 19.7 ± 1.9 | 3.5 ± 0.6 | — |
| | 50 | 16.4 ± 1.9 | 17.2 ± 1.9 | — | — |
| | 25 | 15.4 ± 1.8 | 14.3 ± 1.8 | — | — |
| | 12.5 | 14.8 ± 1.4 | 12.9 ± 1.5 | — | — |
| | 6.2 | 13.5 ± 1.0 | 8.4 ± 1.0 | — | — |
| | 3.1 | 9.1 ± 1.0 | 3.1 ± 0.5 | — | — |
| | 1.6 | 7.6 ± 0.6 | | | |
| PPD | 100 | 8.9 ± 1.2 | 12.0 ± 1.2 | 3.1 ± 0.3 | — |
| | 50 | 7.6 ± 0.8 | 8.3 ± 1.0 | 2.1 ± 0.3 | — |
| | 25 | 4.1 ± 0.3 | 7.1 ± 0.8 | — | — |
| | 12.5 | 2.2 ± 0.3 | 4.3 ± 0.6 | — | — |

**Claims**

1. A proteinaceous substance recognisable by human serum antibodies to *Mycobacterium tuberculosis*, said proteinaceous substance consisting substantially of proteins of molecular weight in the range 5—18 K Daltons, which proteins are immunochemically stable at 80°C and within the pH range 5—8, are substantially free of lipid and carbohydrate residues and contain less than 0.1% diaminopimelic acid.

2. An immunoassay method for the detection of mycobacteria, mycobacterial antigens or antibodies to mycobacterial antigens, the immunoassay comprising the use, as an immunoreactive component, of the proteinaceous substance of claim 1 and/or an antibody or antibodies thereto.

3. An immunoassay method according to claim 2 for the detection of antibodies to *M. tuberculosis* in a test sample, which method comprises steps of a) bringing the test sample into contact with a solid phase to which is bound the proteinaceous substance; b) adding a conjugate comprising an enzyme linked to a substance selectively bindable to the said antibodies and not bindable to the said proteinaceous substance; c) adding substrate to the enzyme, and determining the activity of any enzyme bound to the solid phase.

4. An immunoassay method according to claim 2 for the detection of antigens of *M.tuberculosis* in a test sample, which method comprises the steps of a) bringing the test sample into contact with a solid phase to which is bound antibody to the proteinaceous substance; b) adding a conjugate comprising an enzyme bound to an antibody to the proteinaceous substance; c) adding substrate for the enzyme and determining the activity of any enzyme bound to the solid phase.

5. A kit for use in an immunoassay method according to claim 2, the kit comprising, as an immunoreactive component, the proteinaceous substance of claim 1 and/or an antibody or antibodies thereto.

6. A kit for use in an immunoassay method according to claim 3, the kit comprising the proteinaceous substance and a conjugate, the conjugate comprising an enzyme linked to a substance bindable to the antibody.

7. A kit for use in an immunoassay method according to claim 4, the kit comprising an antibody to the proteinaceous substance and a conjugate, the conjugate comprising an enzyme bound to an antibody to the purified proteinaceous substance.

8. An agglutination test method for the detection of *M. tuberculosis* antigens, or antibodies thereto, in a test sample, which method comprises bringing the test sample into contact with a suspension of particles coated either with the proteinaceous substance of claim 1, or with antibodies thereto, and establishing whether agglutination of the particles has taken place.

9. An agglutination test method according to claim 8 wherein the particles of the suspension are coated with antibodies to the proteinaceous substance.

10. An agglutination test kit for use in the method of claim 8, the kit comprising a suspension of particles coated with the proteinaceous substance.

11. An agglutination test kit for use in the method of claim 9, the kit comprising a suspension of particles coated with antibodies to the proteinaceous substance.

12. A solid phase to which is bound a proteinaceous substance according to claim 1 or an antibody thereto.

13. A conjugate comprising an enzyme linked to an antibody to the proteinaceous substance of claim 1.

14. A composition for use in a skin reactivity test for mycobacterial infection in a patient, the composition comprising the proteinaceous substance of claim 1, a carrier therefor and optionally any other accessory ingredients.

15. A method for the preparation of a proteinaceous substance according to claim 1, which process comprises extraction of mycobacteria with sodium dodecyl sulphate followed by chromatographic removal of excess detergent, and optional further purification by ion-exchange chromatography.


**Patentansprüche**

1. Proteinartige Substanz, die durch humane Serum-Antikörper gegen Mycobacterium tuberculosis erkennbar ist und im wesentlichen aus Proteinen mit einem Molekulargewicht im Bereich von 5 bis 18 K Dalton besteht, wobie die Proteine bei 80°C und innerhalb des pH-Bereiches von 5 bis 8 immunchemisch stabil sind und die im wesentlichen frei von Fett- und Carbohydratresten sind und weniger als 0,1% Diaminopimelinsäure enthalten.

2. Immunoassay-Methode zum Nachweis von Mycobacterien, mycobacteriellen Antigenen oder Antikörper gegen mycobacterielle Antigene, bei der als immunreaktive Komponente die proteinartigen Substanz nach Anspruch 1 und/oder ein Antikörper oder Antikörper dagegen, verwendet wird.

3. Immunoassay-Methode nach Anspruch 2 zum Nachweis von Antikörpern gegen M. tuberculosis in einer Testprobe, bei der man a) die Testprobe mit einer Festphase, an die die proteinartige Substanz gebunden ist in Kontakt bringt; b); ein Konjugat, das ein Enzym, gebunden an eine Substänz, die selektiv an die Antikörper bindbar und nicht bindbar an die proteinartige Substanz ist, zugibt; c) ein Substrat zum Enzym zugibt und die Aktivität eines an die Festphase gebundenen Enzyms bestimmt.

4. Immunoassay-Methode nach Anspruch 2 zum Nachweis von Antigegen von M. tuberculosis in einer Testprobe, bei der man a) die Testprobe mit einer Festphase, an die Antikörper gegen die proteinartige Substanz gebunden sind, in Kontakt bringt; b) ein Konjugat, das ein, an Antikörper gegen die proteinartige Substanz gebundenes Enzym, enthält, zugibt; c) ein Substrat für das Enzym zugibt und die Aktivität eines an die Festphase gebundenen Enzyms bestimmt.

5. Kit zur Verwendung in einer Immunoassay-Methode nach Anspruch 2, wobei der Kit als eine immunreaktive Komponente die proteinartige Substanz nach Anspruch 1 und/oder einen Antikörper oder Antikörper dagegen enthält.

6. Kit zur Verwendung in einer Immunoassay-Methode nach Anspruch 3, wobei der Kit die proteinartige Substanz und ein Konjugat enthält, das ein an eine Substanz, die an den Antikörper bindbar ist, gebundenes Enzym enthält.

7. Kit zur Verwendung in einer Immunoassay-Methode nach Anspruch 4, wobei der Kit einen Antikörper gegen die proteinartige Substanz und ein Konjugat enthält, das ein an einen Antikörper gegen die gereinigte proteinartige Substanz gebundenes Enzym enthält.

8. Agglutinationstest-Methode zum Nachweis von M. tuberculosis Antigenen oder Antikörper dagegen in einer Testprobe, bei der man die Testprobe mit einer Suspension von Partikeln, die entweder mit der proteinartigen Substanz nach Anspruch 1 oder mit Antikörpern dagegen, beschichtet sind in Kontakt bringt und feststellt, ob eine Agglutination der Partikel stattgefunden hat.

9. Agglutinationstest-Methode nach Anspruch 8, bei der die Partikel der Suspension mit Antikörper gegen die proteinartige Substanz beschichtet sind.

10. Agglutinationstest-Kit zur Verwendung in der Methode nach Anspruch 8, wobei der Kit eine Suspension von·Teilchen enthält, die mit der proteinartigen Substanz beschichtet sind.

11. Agglutinationstest-Kit zur Verwendung in der Methode nach Anspruch 9, wobei der Kit eine Suspension von Teilchen enthält, die mit Antikörpern gegen die proteinartige Substanz beschichtet sind.

12. Festphase, an die ein proteinartige Substanz nach Anspruch 1 oder ein Antikörper dagegen gebunden ist.

13. Konjugat, enthaltend ein Enzym, gebunden an einen Antikörper gegen die proteinartige Substanz nach Anspruch 1.

14. Zusammensetzung zur Verwendung in einem Hautreaktionstest für mycobacterielle Infektionen in

24

# EP 0 174 805 B1

einem Patienten, wobei die Zusammensetzung die proteinartige Substanz nach Anspruch 1, einen Träger und gegebenenfalls andere Hilfsmittel enthält.

15. Verfahren zur Herstellung einer proteinartigen Substanz nach Anspruch 1, bei der man Mycobacterien mit Natriumdodecylsulfat extrahiert, anschließend das überschüssige Detergens chromatographisch entfernt und gegebenenfalls durch Ionenaustauschchromatographie weiterreinigt.

## Revendications

1. Substance protéique qui peut être reconnue par des anticorps du sérum humain dirigés contre *Mycobacterium tuberculosis,* ladite substance protéique consistant principalement en protéines de poids moléculaire de l'ordre de 5 à 18 Kdaltons, lesdites protéines étant immunochimiquement stables à 80°C et dans la gamme de pH de 5 à 8, ces protéines étant essentiellement exemptes de résidus lipidiques et d'hydrates de carbone et contenant moins de 0,1% d'acide diaminopimélique.

2. Méthode de dosage immunologique pour la détection des mycobactéries, des antigènes mycobactériens ou des anticorps dirigés contre les antigènes mycobactériens, ladite méthode d'immunodétection comprenant l'utilisation, comme constituant immunoréactif, de la substance protéique selon la revendication 1 et/ou d'un ou plusieurs anticorps dirigés contre celle-ci.

3. Méthode de dosage immunologique selon la revendication 2, pour la détection d'anticorps contre *M. tuberculosis* dans un échantillon à tester, ladite méthode comprenant les étapes suivantes: a) mise en contact de l'échantillon à tester avec une phase solide à laquelle est liée la substance protéique; b) addition d'un conjugué comprenant une enzyme liée à une substance qui peut sélectivement être liée auxdits anticorps et qui ne peut pas se lier à ladite substance protéique; c) addition du substrat de l'enzyme et détermination de l'activité de toute enzyme fixée à la phase solide.

4. Méthode de dosage immunologique selon la revendication 2, pour la détection d'antigènes de *M. tuberculosis* dans un échantillon à tester, ladite méthode comprenant les étapes suivantes: a) mise en contact de l'échantillon à tester avec une phase solide à laquelle est lié un anticorps dirigé contre la substance protéique; b) addition d'un conjugué comprenant une enzyme liée à un anticorps dirigé contre la substance protéique; c) addition du substrat de l'enzyme et détermination de l'activité de toute enzyme liée à la phase solide.

5. Trousse pour l'utilisation de la méthode de dosage immunologique selon la revendication 2, ladite trousse comprenant, comme constituant immunoréactif, la substance protéique selon la revendication 1 et/ou un ou plusieurs anticorps dirigés contre celle-ci.

6. Trousse pour l'utilisation de la méthode de dosage immunologique selon la revendication 3, ladite trousse comprenant la substance protéique et un conjugué, ledit conjugué comprenant une enzyme liée à une substance qui peut être liée à l'anticorps.

7. Trousse pour l'utilisation de la méthode de dosage immunologique selon la revendication 4, ladite trousse comprenant un anticorps dirigé contre la substance protéique et un conjugué, ledit conjugué comprenant une enzyme liée à un anticorps dirigé contre la substance protéique purifiée.

8. Méthode basée sur un test d'agglutination pour la détection d'antigènes de *M. tuberculosis* ou d'anticorps dirigés contre ceux-ci, ladite méthode consistant à mettre en contact l'échantillon à tester avec une suspension de particules recouvertes soit de la substance protéique selon la revendication 1, soit des anticorps dirigés contre celle-ci, et à déterminer si l'agglutination des particules se produit.

9. Méthode basée sur un test d'agglutination selon la revendication 8, dans laquelle les particules de la suspension sont recouvertes d'anticorps dirigés contre la substance protéique.

10. Trousse pour test d'agglutination pour l'utilisation de la méthode selon la revendication 8, ladite trousse comprenant une suspension de particules recouvertes de la substance protéique.

11. Trousse pour test d'agglutination pour l'utilisation de la méthode selon la revendication 9, ladite trousse comprenant une suspension de particules recouvertes d'anticorps dirigés contre la substance protéique.

12. Phase solide à laquelle est liée une substance protéique selon la revendication 1 ou un anticorps dirigé contre celle-ci.

13. Conjugué comprenant une enzyme liée à un anticorps dirigé contre la substance protéique selon la revendication 1.

14. Composition destinée à l'utilisation en test de réactivité cutanée pour les infections mycobactériennes chez un patient, ladite composition comprenant la substance protéique selon la revendication 1, un support pour celle-ci et éventuellement tout autre ingrédient accessoire.

15. Méthode pour la préparation de la substance protéique selon la revendication 1, ladite méthode comprenant l'extraction au dodécylsulfate de sodium à partir des mycobactéries, suivie de l'élimination par chromatographie de l'excès de détergent et, éventuellement, une purification supplémentaire par chromatographie par échange d'ions.